# EUROPEAN PATENT APPLICATION

(11) **EP 4 653 029 A1**
(43) Date of publication of application: **26.11.2025**
(21) Application number: 25174717.6
(22) Date of filing: 07.05.2025
(51) Int. Cl.: A61M 5/178, A61F 9/00, A61M 5/32

(54) **INJECTION DEVICE WITH STABILIZING PASSIVE NEEDLE SHIELD**

(30) Priority: 21.05.2024 US 202418669772
(71) Applicant: Becton, Dickinson and Company, Franklin Lakes, NJ 07417-1880 (US)
(72) Inventor: RAO, P. Preran, 560076 Bengaluru, Karnataka (IN); B., Srinath, 560045 Bengaluru, Karnataka (IN); MR, Karthik, 560067 Bengaluru, Karnataka (IN); PRASAD, Shishir, Ramsey, NJ, 07446 (US); DHARMADHIKARI, Radhika, 411038 Pune (IN)
(74) Representative: dompatent

(57) **Abstract**

Medical injection devices incorporate collapsible, passive needle shields for avoiding inadvertent needle tip contact with objects. The needle shield is coupled to the distal end of the syringe barrel, enveloping the needle in its relaxed, non-compressed state. When the needle shield is in a relaxed, non-collapsed state, its contact surface extends distally longer than the needle tip, so that the tip is enveloped and shielded by the contact surface. Abutment of the contact surface against a desired patient body site stabilizes the syringe orientation. Thereafter, the syringe barrel is advanced, which passively collapses the needle shield, so that the now exposed needle tip pierces the patient's body, allowing injection of medical fluids by advancement of the syringe plunger. Injection device embodiments include ocular injection devices for injecting medication through the sclera of a patient's eye.

## Description

### TECHNICAL FIELD

The present disclosure generally relates to injection devices, including syringes, for injecting medicinal fluids into patients, with stabilizing needle shields for avoiding inadvertent needle tip contact with users, patients or other objects. More particularly, embodiments of the present disclosure relate to injection devices with passive needle shields that do not require activation by the administering medical practitioner, for avoiding inadvertent needle tip contact with the practitioner, patient or other unintended objects while also providing stability at the injection site. Exemplary embodiments of the present disclosure relate to ocular injection devices.

### BACKGROUND

Drug injection devices, such as dispensing syringes are generally packaged with a separable needle shield covering the sharp tip of the injection needle to prevent inadvertent contact with objects, often referred to as "needle sticks," during the entire syringe use cycle. Separable needle shields are also used to cover independently packaged injection needles that are intended for coupling to syringes prior to drug dispensing. Generally, the syringe use cycle starts at injection device/dispensing needle supply chain steps of manufacture, packaging, shipping to and storage at a medical facility, clinical use of the injection device/dispensing needle to treat a patient, and their medical waste disposal.

During clinical practice, the need to remove a separate, external needle shield during drug administration with the syringe/needle increases clinician time and risks potential loss of needle sterility during its exposure to ambient air or contact with non-sterile objects. Replacement of the external needle shield on the now spent syringe/needle exposes the clinician to a needle stick during the replacement and expends more clinician time. If clinical procedures mandate direct placement of the spent needle syringe into a disposal receptacle for medical waste without replacing the external needle shield, the clinician and others potentially risk a needle stick during transport of the used syringe to the disposal receptacle. Some syringe designs incorporate integral needle shields where a mechanically translatable needle is advanced distally out of the syringe by clinician active manipulation of a needle translation mechanism.

Certain injection practices, such as ocular injections, dermatological injections, neonatal injection, injection of biologics, or spinal injections, add additional complex clinical challenges compared to routine injection through the patient's dermal layer on an upper arm or the like. For such injection practices, delivery of the injection may require precise administration or administration to sensitive sites. For instance, some patients may be susceptible to involuntary eye and eyelid movements during ocular injections. In order to administer an intra-ocular injection to a desired injection site, such as the sclera, without inadvertently damaging the sclera or cornea requires eye immobilization. One known way to attempt eye immobilization is by asking the patient to roll the eye to one side to expose sclera tissue as distant as possible from the cornea, with the hope that the intentional eye roll will overcome involuntary eye movement. Another known immobilization method is utilization of an external forceps-type device that surrounds the cornea to restrict involuntary eye movement. Even when these known eye immobilization techniques are successful, there remains difficulty aligning and/or stabilizing the freestanding, unsupported needle tip at the injection site at a desired insertion angle relative to the sclera surface and potential sclera tearing if there is eye movement relative to the needle tip during the actual tissue puncture. Similar clinical challenges exist for certain dermatological administrations, neonatal injections, biologic injections, spinal injections, and others.

There is a need for an injection device, such as a syringe, which has the means to shield its dispensing needle tip passively during preparation for patient dosing, alignment of the syringe with a dosing site on the patient's body immediately preceding needle puncture, withdrawal of the injection device from the patient and disposal of the spent syringe. There is also a need for an injection device with a passive needle shield, for drug administration, for easier alignment and stability of the needle tip immediately prior to injection, while reducing potential injuries to the patient and clinicians during injection, for increasing clinician efficiency, and for reducing costs associated with medical waste disposal. One specific clinical need is for an ocular injection device with a passive needle shield, for intra-ocular drug administration through the patient's sclera.

### SUMMARY

The present disclosure is directed to injection devices, such as syringes, with passive needle shields that do not require removal and disposal of an external needle shield or activation of a needle shield feature by a healthcare provider who is administering a medical fluid dosage to a patient. The passive needle shields avoid inadvertent needle tip contact with objects, including for example the patient or medical practitioners who are treating the patient. In some embodiments, the syringes with passive needle shields are ocular syringes for injecting fluids through the sclera of a patient's eye while avoiding inadvertent needle tip contact with the practitioner, patient or other unintended objects. The passive needle shield surrounds the syringe's needle tip without any intentional action of the medical practitioner who is dosing a delivery site of the patient, including but not limited to intra-ocular. A collapsible needle shield is coupled to the distal end of the syringe barrel. The needle shield circumscribes the needle. The needle shield has a contact surface on its distal axial end with an aperture that is coaxial with the needle. When the needle shield is in a relaxed, non-collapsed state, its contact surface extends distally longer than the needle tip, so that the tip is shielded by the contact surface. After the medical practitioner abuts the contact surface against a desired position on the patient, thereby stabilizing the injection device relative to the patient's tissue at the desired injection site, the syringe barrel is advanced, which passively collapses the needle shield, so that the now exposed needle tip pierces the desired injection site, allowing injection of medical fluids into the desired location of the patient. In some embodiments, the maximum collapsed length of the needle shield limits needle insertion depth.

In a specific embodiment, when the contact surface contacts the patient's sclera at a desired injection site, the distal tip of the shield and the entire injection device is stabilized relative to and moves with the eye. When the needle shield is in a relaxed, non-collapsed state, its contact surface extends distally longer than the needle tip, so that the tip is shielded by the contact surface. After the medical practitioner abuts the contact surface against a desired position on the patient's sclera, the injection device is stabilized; the syringe barrel is advanced, which passively collapses the needle shield, so that the now exposed needle tip pierces the sclera at the desired injection site, allowing injection of medical fluids into the patient's eye. In some embodiments, the maximum collapsed length of the needle shield limits needle insertion depth, for example to avoid needle hub contact with the sclera.

One aspect of the present disclosure pertains to an injection device, comprising a syringe including a barrel that defines a barrel cavity with a proximal open end, a barrel tip on a distal end of the barrel, and a plunger inserted in a proximal open end of the barrel that is translatable in the barrel cavity. The plunger defines a plunger stopper on its distal end. A needle hub is coupled to the barrel tip; it is coupled to a needle that defines a needle tip that is oriented distal the needle hub. The needle extends a first axial length relative to the needle hub. The barrel cavity, barrel tip, needle hub and needle are in fluid communication with each other. The injection device also has a collapsible needle shield coupled to the distal end of the syringe barrel, with the needle shield extending distally from the needle hub and circumscribing the needle. In some embodiments, the needle shield is coupled to the needle hub. The needle shield has a contact surface on its distal axial end for abutment against and stabilization of the injection device at a desired injection site of the patient and an aperture formed therein that is coaxial with the needle. When the needle shield is in a relaxed, non-collapsed state, its contact surface extends axially from the needle hub a second axial length that is longer than the first axial length of the needle, so that the needle tip is oriented proximal to and shielded by the contact surface. When the needle shield is in a collapsed state, its contact surface extends axially from the needle hub a third axial length that is shorter than the first axial length of the needle, so that the needle tip is exposed and extends distally through the aperture of the flexible contact surface. In some embodiments, the contact surface is an ocular contact surface with a surface profile for abutment against a sclera of a patient's eye. In some embodiments, the contact surface is flexible and conforms to the surface profile of the patient's tissue, such as the sclera.

An additional aspect of the present disclosure pertains to an injection device, comprising a syringe including a barrel that defines a barrel cavity with a proximal open end, a barrel tip on a distal end of the barrel, and a plunger inserted in a proximal open end of the barrel that is translatable in the barrel cavity. The plunger defines a plunger stopper on its distal end. A needle hub is coupled to the barrel tip; it is coupled to a needle that defines a needle tip that is oriented distal the needle hub. The needle extends a first axial length relative to the needle hub. The barrel cavity, barrel tip, needle hub and needle are in fluid communication with each other. The injection device also has a flexible bellows tube forming a collapsible needle shield, the bellows tube defining an internal cannula. The proximal axial end of the bellows tube is coupled to the needle hub. The bellows tube extends distally from the needle hub and circumscribes the needle within its internal cannula. A distal axial end of the bellows tube is coupled to a cup-shaped skirt, whose distal axial end defines a contact surface, and an aperture formed in the cup-shaped skirt that is in communication with the internal cannula and that is coaxial with the needle. When the flexible bellows is in a relaxed, non-collapsed state, the contact surface of the skirt extends axially from the needle hub a second axial length that is longer than the first axial length of the needle, so that the needle tip is oriented proximal to and shielded by the contact surface. When the flexible bellows is in a collapsed state, the contact surface of the skirt extends axially from the needle hub a third axial length that is shorter than the first axial length of the needle, so that the needle tip is exposed and extends distally through the aperture of the contact surface.

Yet another aspect of the present disclosure pertains to an injection device, comprising a syringe including a barrel that defines a barrel cavity with a proximal open end, a barrel tip on a distal end of the barrel, and a plunger inserted in a proximal open end of the barrel that is translatable in the barrel cavity. The plunger defines a plunger stopper on its distal end. A needle hub is coupled to the barrel tip; it is coupled to a needle that defines a needle tip that is oriented distal the needle hub. The needle extends a first axial length relative to the needle hub. The barrel cavity, barrel tip, needle hub and needle are in fluid communication with each other. The injection device also incorporates a collapsible needle shield, including a needle shield hub coupled to the needle hub. First and second hinge assemblies, each respectively having a proximal link, are coupled to the needle shield hub, a link hinge coupled to a distal end of the proximal link, and a distal link coupled to the link hinge distal the proximal link. The collapsible needle shield also has a contact surface, which in some embodiments is an ocular contact surface, coupled to the respective distal ends of the distal links of the first and second hinge assemblies. The contact surface defines a through aperture that is oriented coaxially with the needle. A tubular cover is coupled to a proximal side of the contact surface and oriented coaxial with the needle; a cannular passage of the tube is in communication with the aperture for passage of the needle therethrough. When the collapsible needle shield is in a relaxed, non-collapsed state, the contact surface extends axially from the needle hub a second axial length that is longer than the first axial length of the needle, so that the needle tip is oriented proximal to and shielded by the contact surface and the tubular cover. When the collapsible needle shield is in a collapsed state, the contact surface extends axially from the needle hub a third axial length that is shorter than the first axial length of the needle, so that the needle tip is exposed and extends distally through the aperture of the contact surface.

An additional aspect of the present disclosure pertains to a method for injecting a with one or more of the above-described injection devices. The method is practiced by contacting a desired site on the patient's body with the contact surface of the injection device. The syringe barrel is advanced to depress the body site with the contact surface, thereby stabilizing the aperture of the contact surface relative to the body site. After stabilizing the aperture, the syringe barrel is pivoted, thereby orienting a central axis of the needle in a desired angular position relative to the body site. Thereafter, further advancing the syringe barrel, collapses the needle shield and pierces the body site with the needle tip to a desired depth within the body. Once the patient's body site is pierced to a desired depth, fluid contained in the syringe barrel's cavity is injected into the patient. After injection is completed, the syringe is withdrawn from the patient's body, whereupon the needle shield relaxes passively from its collapsed state and again shields the needle tip from inadvertent contact with the patient or any other object.

A further aspect of the present disclosure pertains to a method for injecting a patient's eye with one or more of the above-described injection devices. The method is practiced by contacting a desired site on a sclera of a patient's eye with the contact surface of the injection device. The syringe barrel is advanced to depress the sclera with the contact surface, thereby stabilizing the aperture of the contact surface relative to the sclera. After stabilizing the aperture, the syringe barrel is pivoted, thereby orienting a central axis of the needle in a desired angular position relative to the sclera. Thereafter, further advancing the syringe barrel, collapses the needle shield and pierces the sclera with the needle tip to a desired depth within the eye. Once the sclera is pierced to a desired depth, fluid contained in the syringe barrel's cavity is injected into the intra ocular region of the patient's eye. After injection is completed, the syringe is withdrawn from the patient's eye, whereupon the needle shield relaxes passively from its collapsed state and again shields the needle tip from inadvertent contact with the patient's eye or any other object.

### BRIEF DESCRIPTION OF THE DRAWINGS

Exemplary embodiments of the disclosure are further described in the following detailed description in conjunction with the accompanying drawings, in which:
FIG. 1 is an isometric view of an embodiment of an injection device with a passive needle shield in a relaxed state, with the needle tip circumscribed by the needle shield;
FIG. 2 is an enlarged view of the passive needle shield portion of the injection device of FIG. 1, showing the needle tip surrounded by the needle shield;
FIG. 3 is an elevational view of the injection device of FIG. 1, aligned with a desired site on the sclera of a patient's eye, in preparation for ocular injection of a medicinal fluid, with the needle shield in a relaxed state;
FIG. 4 is an elevational view of the injection device of FIG. 1 during intra-ocular injection of the medicinal fluid into the patient, with the needle shield collapsed and the needle tip penetrating the sclera;
FIG. 5 is an isometric view of another embodiment of an injection device with a passive needle shield, in a relaxed state, with the needle tip circumscribed and enveloped by the needle shield;
FIG. 6 is an enlarged view of the passive needle shield portion of the injection device of FIG. 5, showing the needle tip enveloped by the needle shield; and
FIG. 7 is an enlarged top plan view of the passive needle shield portion of the injection device of FIG. 5, showing the needle shield in a collapsed position, with the needle tip exposed.

To facilitate understanding, identical reference numerals have been used, where possible, to designate identical elements that are common to the figures. The figures are not drawn to scale.

### DETAILED DESCRIPTION

Injection devices, such as syringes, include passive needle shields that do not require activation by a healthcare provider who is administering a medical fluid dosage to a patient. The passively active needle shields avoid inadvertent needle tip contact with objects (sometimes referred to as "needle sticks"), including for example the patient or medical practitioners who are treating the patient. In some embodiments, the syringes are ocular syringes for injecting fluids through the sclera of a patient's eye that incorporate passive needle shields for avoiding inadvertent needle tip contact with objects. In other embodiments, the injection devices are utilized for dermatological injection, neonatal injection, injection of biologics, or spinal injection that may require precise administration or administration to sensitive tissue sites. A collapsible needle shield is coupled to the distal end of the syringe barrel. The needle shield circumscribes the needle. The needle shield has a contact surface on its distal axial end for abutment and stabilization against patient tissue at a desired injection site The contact surface has an aperture that is coaxial with the needle. When the needle shield is in a relaxed, non-collapsed state, its contact surface extends distally longer than the needle tip, so that the tip is shielded by the contact surface. After abutment of the contact surface against a desired site on the patient's body, thereby stabilizing the injection device, the syringe barrel is advanced, which passively collapses the needle shield, so that the now exposed needle tip pierces patient tissues at that site, allowing injection of medical fluids into the patient's body. In some embodiments, the injection device is an ocular injection device, wherein the contact surface is an ocular contact surface having a profile for abutment against the sclera of a patient's eye. While many embodiments of the injection devices with passive needle shields disclosed herein are for ocular applications, they are also suitable for administration of medical fluids to other parts of a patient's body, such as for performing dermatological injections, neonatal injections, injection of biologics, or spinal injections. In some of these other applications, the profile of the needle shield's contact surface is modified to conform to the desired body part. In some applications the contact surface is flat.

In this disclosure, a convention is followed wherein the distal end of the device is the end closest to a patient, e.g., for delivery of one or more drugs to the patient, and the proximal end of the device is the end away from the patient and closest to a clinician or other medical practitioner. With respect to terms used in this disclosure, the following definitions are provided.

As used herein, the use of "a," "an," and "the" includes the singular and plural.

As used herein, the term "Luer connector" refers to a connection collar that is the standard way of attaching syringes, catheters, hubbed needles, I.V. tubes, etc. to each other. The Luer connector consists of male and female interlocking tubes, slightly tapered to hold together better with even just a simple pressure/twist fit. Luer connectors can optionally include an additional outer rim of threading, allowing them to be more secure. The Luer connector male end is associated with a syringe discharge tip and can interlock and connect to the female end that is incorporated within a proximal end of a needle hub of a drug dispensing needle or filling needle for aspirating a drug from a drug vial into a syringe. Luer connector female ends are commonly located on vascular access devices (VADs).

As used herein, ISO 80369-7:2016 defines a specification for standard Luer connectors including a 6% taper between the distal end and the proximal end. A male standard Luer connector increases from the open distal end to the proximal end. A female standard Luer connector decreases from the open proximal end to the distal end. According to ISO 80369-7:2016, a male standard Luer connector has an outer cross-sectional diameter measured 0.75 mm from the distal end of the tip of between 3.970 mm and 4.072 mm. The length of the male standard Luer taper is between 7.500 mm to 10.500 mm. The outer cross-sectional diameter measured 7.500 mm from the distal end of the tip is between 4.376 mm and 4.476 mm. As used herein, the phrases "male standard Luer connector" and "female standard Luer connector" shall refer to connectors having the dimensions described in ISO 80369-7, which is hereby incorporated by reference in its entirety.

As would be readily appreciated by skilled artisans in the relevant art, while descriptive terms such as "tip", "hub", "thread", "protrusion/insert", "tab", "wall", "top", "side", "bottom" and others are used throughout this specification to facilitate understanding, it is not intended to limit any components that can be used in combinations or individually or to require specific spatial orientations, to implement various aspects of the embodiments of the present disclosure.

Before describing several exemplary embodiments of the disclosure, it is to be understood that the disclosure is not limited to the details of construction or process steps set forth in the following description. The disclosure is capable of other embodiments and of being practiced or being conducted in many ways.

The matters exemplified in this description are provided to assist in a comprehensive understanding of exemplary embodiments of the disclosure. Accordingly, those of ordinary skill in the art will recognize that various changes and modifications of the embodiments described herein can be made without departing from the scope and spirit of the disclosure. Also, descriptions of well-known functions and constructions are omitted for clarity and conciseness.

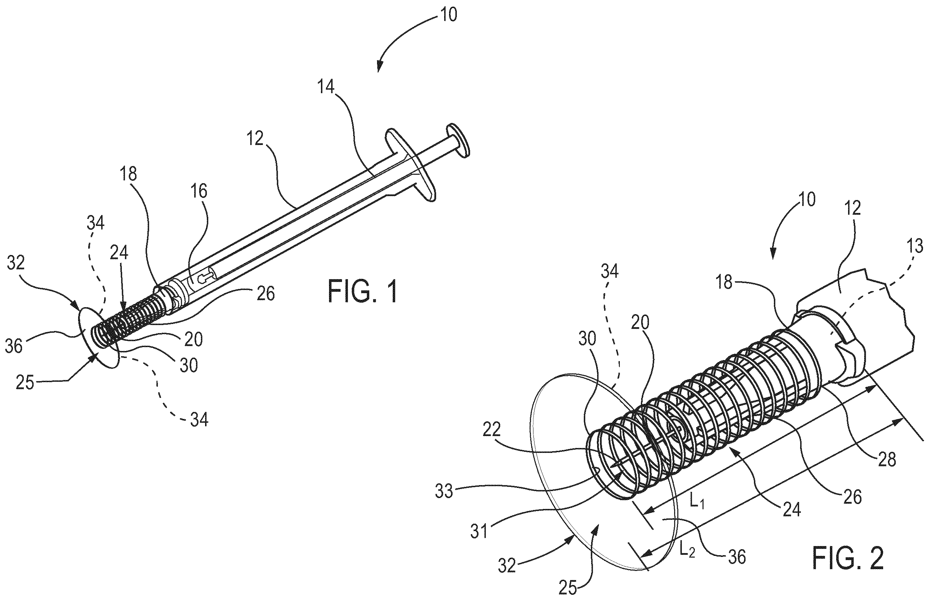

The following non-limiting examples demonstrate principles according to one or more embodiments of the disclosure. FIGs. 1 and 2 depict a first embodiment of a medical injection device or syringe 10, with a barrel 12 that defines an internal barrel cavity with a proximal open end and a barrel tip 13 on a distal end of the barrel. In some embodiments, the barrel tip comprises a Luer connector. A plunger 14 is inserted in the proximal open end of the barrel and is translatable in the barrel cavity. The plunger 14 has a plunger stopper 16 on its distal end. A dosage of medical fluid is contained in the barrel cavity between the barrel tip 13 and the distal end of the plunger stopper 16. The proximal end of needle hub 18 is coupled to the barrel tip 13. In some embodiments the barrel tip comprises a Luer connector, and the proximal end of the needle hub has a mating Luer connector. The distal end of the needle hub 18 is coupled to a proximal end of a dosing needle 20 whose distal axial end defines a needle tip 22. The needle 20 extends a first axial length L₁ between the needle hub 18 and the needle tip 22. As is common with medicinal syringes, such as the injection device 10, the barrel cavity, barrel tip 13, needle hub 18 and the hollow needle 20 are in fluid communication with each other for administration of medicinal fluids through the needle tip 22 into a patient at a desired delivery site.

A collapsible needle shield 24 is coupled to the distal end of the syringe barrel 12. In the embodiment of FIGs. 1 and 2 the needle shield 24 is directly coupled to the outer circumference of the needle hub 18. In other embodiments, the needle shield is coupled to the outer circumference of the syringe barrel. In other embodiments the needle shield is formed integrally with the needle hub. As shown in FIGs. 1 and 2, the needle shield 24 extends distally from the needle hub 18 and terminates at an axial length L₂ between the needle hub 18 and a contact surface 25. Thus, the needle shield 24 envelops the needle 20 and the needle tip 22 both circumferentially and axially, inhibiting inadvertent contact or other needle sticks with other objects, such as clinicians or the patient. As shown in those figures, the collapsible needle shield 24 is in its relaxed, uncompressed, non-collapsed state; the needle tip 22 remains shielded passively until a clinician commences patient injection. In other words, when the needle shield 24 is in its relaxed, non-collapsed state, its distal contact surface 25 extends axially from the needle hub 18 the second axial length L₂ that is longer than the first axial length L₁ of the needle 20, so that the needle tip 22 is oriented proximal to and shielded by the contact surface 25.

The injection device 10 embodiment of FIGs. 1-4 is suitable for injection of patients, including but not limited to ocular site injection, dermatological administrations, neonatal injection, biologics injection, spinal injection, and others. In this embodiment, the collapsible needle shield 24 is a flexible, corrugated bellows tube 26, having a length L₂, thus shielding the needle tip 22 when it is in a relaxed, non-collapsed state, The contact surface of the bellows tube 26 functions as a tissue contact surface. The bellows tube 26 has a maximum collapsible length L₃ for setting maximum penetration depth of the needle tip 22 and/or preventing bottoming out of distal end of the needle hub 18 against patient body tissue. A proximal axial end 28 of the bellows tube 26 is directly coupled to the needle hub 18, such as by shrink or stretched fit. The distal axial end 30 of the bellows tube 26 defines the contact surface 25. The bellows tube 26 defines an internal cannula 31 that envelops or otherwise circumscribes the needle 20 and the needle tip 22 both circumferentially and axially when in a relaxed state.

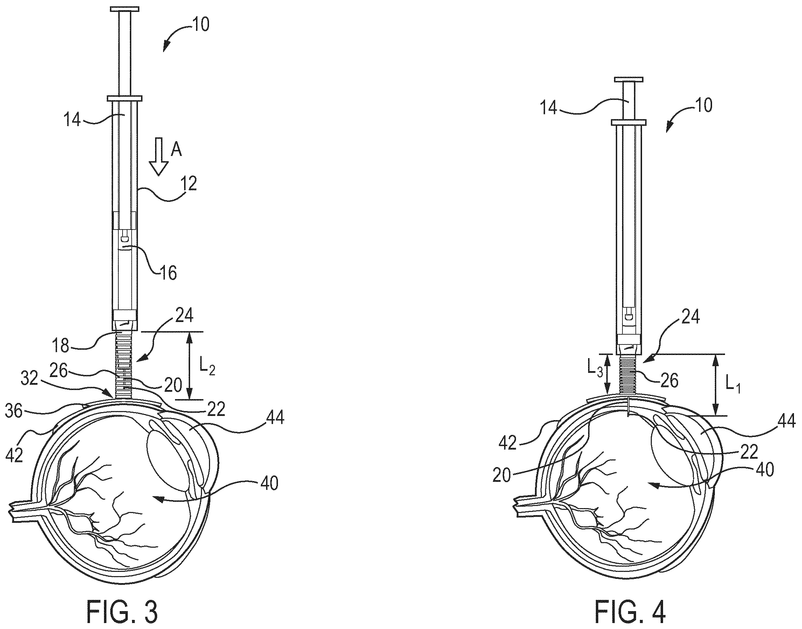

In the injection device 10 embodiment of FIGs. 1-4, the functioning contact surface 25 is a flexible cup-shaped skirt 32 with a bellows aperture 33 that is in communication with the internal cannula 31 and that is coaxial with the needle 20, for passage of the needle tip 22 therethrough when the bellows tube is collapsed sufficiently so that its collapsed length L₃ is shorter than the needle length L₁. The skirt 32 has a proximal face 34 and a distal face 36 for compliant, abutting contact with patient tissue, for example a sclera of a patient's eye 40. In FIGs. 3 and 4, the distal face 36 of the skirt 32 is abutted against a desired injection site on the sclera 42 of the patient's eye 40 proximal the cornea 44. In some embodiments, the compliant, cup-shaped skirt 32 adheres to and/or suction anchors to the sclera 42 or to other body tissue. The flexible bellows tube 26 and a cup-shaped, flexible skirt 32 are formed as a polymer unistructure, such as a silicon hydrogel polymer unistructure. In some embodiments, the bellows tube and the flexible skirt are separate components that are cojoined. In some embodiments, the contact surface 25 is not flexible. In some embodiments, the bellows tube 26 and the flexible skirt 32 are transparent or translucent, for visible verification of the orientation of the shielded needle tip 22. In some embodiments, the injection device incorporates a flexible bellows tube. In some embodiments, the passive needle shield and the contact surface are transparent or translucent.

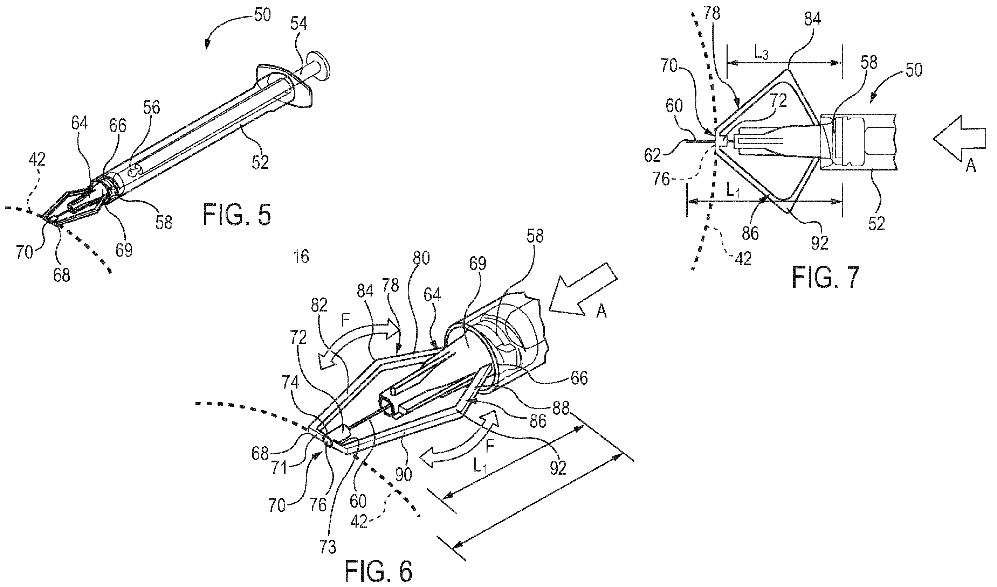

FIGS 5-7 depict a second embodiment of an injection device 50 of the present disclosure. The injection device or syringe 50 is suited for ocular injection as well as for injection of other body tissue of a patient, such as for dermatological administrations, neonatal injection, biologics injection, spinal injection, and others. The syringe 50 includes a barrel 52 that defines a barrel cavity with a proximal open end, a barrel tip on a distal end of the barrel, and a plunger 54 inserted in a proximal open end of the barrel that is translatable in the barrel cavity. The plunger includes a plunger stopper 56 on its distal end. A needle hub is 58 coupled to the barrel tip; it is coupled to a needle 60 that defines a needle tip 62, which is oriented distal the needle hub. The needle 60 extends a first axial length L₁ relative to the needle hub 58. The barrel cavity, barrel tip, needle hub 58 and needle 60 are in fluid communication with each other.

The injection device 50 also incorporates a collapsible needle shield 64 having a proximal axial end 66 and a distal axial end 68. The proximal axial end 66 includes needle shield hub 69, which is directly coupled to the needle hub 58. In other embodiments, the needle shield is coupled to the outer circumference of the syringe barrel. In other embodiments the needle shield is formed integrally with the needle hub. Needle shield 64 has a distal end 68 that terminates in a contact surface. In the embodiments of FIGs. 5-7, the contact surface 70 is a curved contact surface with a distal face 71 whose profile that is adapted for abutment against an eye sclera. In other embodiments, the surface profile of the contact surface 70 is adapted for abutment and stabilization against other types of body tissue. A tubular cover 72 is coupled to a proximal side 73 of the contact surface 70; the tubular cover is oriented coaxial with the needle 60. A cannular passage 74 of the tubular cover 72 is in communication with a through aperture 76 formed within the proximal face 71 of the contact surface 70, for passage of the needle 60 and needle tip 62 when the needle shield is in a collapsed state, such as shown in FIG. 7. When the needle shield 68 is in its relaxed, non-collapsed state of FIG. 6, the needle tip 62 is shielded within the tubular cover 72 in a functional fashion analogous to the relaxed, non-collapsed state of the needle shield 24 of the syringe 10 of FIG. 2.

With reference to FIG. 6, the needle shield 64 has a first 78 and a second 86 knee-like, collapsible hinge assembly. The first hinge assembly 78 has a first proximal link 80 coupled to the needle shield hub 69, a first link hinge 84 coupled to a distal end of the first proximal link 80, and a first distal link 82 coupled to the first link hinge 84 distal the first proximal link 80. Similarly, the second hinge assembly 86 has a second proximal link 88 coupled to the needle shield hub 69, a second link hinge 92 coupled to a distal end of the second proximal link 88, and a second distal link 90 coupled to the second link hinge 92 distal the second proximal link 88. The contact surface 70 is coupled to the respective distal ends of the first 82 and second 90 distal links of the first 78 and second 86 hinge assemblies.

When the collapsible needle shield is in the relaxed, non-collapsed state of FIGs. 5 and 6, the contact surface 70 extends axially from the needle hub 58 a second axial length L₂ that is longer than the first axial length L₁ of the needle 60, so that the needle tip is oriented proximal to and shielded by the contact surface 70 and the tubular cover 72. In FIG. 7, when the collapsible needle shield is in a collapsed state, by application of the advancement force A to flex F the first 84 and second 92 hinges (see double arrows A and F of FIG. 6) the contact surface 70 extends axially from the needle hub 58 a third axial length L₃ that is shorter than the first axial length of the needle, so that the needle tip 62 is exposed and extends distally through the aperture of the contact surface 70, penetrating the sclera 42. In some embodiments, the third axial length L₃ is a maximum collapsible length for setting maximum penetration depth of the needle tip 62 and/or preventing bottoming out of distal end of the needle hub 58 against patient body tissue. In the embodiment of FIGs. 5-7, the designed range of angular motion of the first 84 and second 92 hinges determines the maximum collapsible length for axial length L₃. In some embodiments, the collapsible needle shield 69 comprises a polymer unistructure.

The injection device or syringe embodiments disclosed herein are constructed from medical grade materials known to one skilled in the art. In some embodiments, described barrels, plungers and shafts are fabricated with polypropylene polymers. Seals, if any, are fabricated with fiber-filled polytetrafluoroethylene (PTFE) polymers. Stoppers are fabricated with polyisoprene polymers.

Various embodiments of the injection devices with passive needle shields described herein are used by clinicians to administer medical fluids to patients. The method is practiced by contacting a desired site on the patient's body with the contact surface of the injection device, which stabilizes the injection device relative to the desired injection site. The syringe barrel is advanced to depress the body site with the contact surface, thereby anchoring or stabilizing the aperture of the contact surface relative to the body site. After stabilizing the aperture, the syringe barrel is pivoted, thereby orienting a central axis of the needle in a desired angular position relative to the body site. Thereafter, further advancing the syringe barrel, collapses the passive needle shield and pierces the body site with the needle tip to a desired depth within the body. Once the patient's body site is pierced to a desired depth, fluid contained in the syringe barrel's cavity is injected into the patient. After injection is completed, the syringe is withdrawn from the patient's body, whereupon the needle shield relaxes passively from its collapsed state and again shields the needle tip from inadvertent contact with the patient or any other object.

An exemplary description of ocular injection methodology is illustrated with the first injection device 10 embodiment of FIGs. 3 and 4. The same ocular injection can be performed with the second embodiment injection device 50 of FIGs. 5-7. A clinician contacts a desired injection site on a sclera 42 of a patient's eye 40 with the contact surface, namely, the distal face 36 of the cup-shaped skirt 32, while the needle tip 22 remains shielded within the needle shield 24 The syringe barrel is advanced (double arrow A) sufficiently to depress the sclera with the distal face 36 of the contact surface, thereby or stabilizing the aperture of the ocular contact surface relative to the sclera. In this way, the desired injection site is established, so that the ocular contact surface moves with the injection site, despite patient involuntary eye movement. Syringe barrel 12 advancement force A that is used to anchor or stabilize the contact surface is below that necessary to collapse the needle shield 24 to expose the needle tip 22. After stabilizing the aperture relative to the sclera 42, the syringe barrel 12 is pivoted, thereby orienting a central axis of the needle 20 in a desired angular position relative to the sclera. Thereafter, further advancing the syringe barrel 12, by application of additional force A, collapses the bellows tube 26 of the needle shield 24 and pierces the sclera 42 with the needle tip 22 to a desired depth within the eye 40 Once the sclera 42 is pierced to a desired depth, medicinal fluid contained in the cavity of the syringe barrel 12 is injected into the intra-ocular region of the patient's eye 40. After injection is completed, the needle 20 of the syringe 10 is withdrawn from the patient's eye 40, whereupon the bellows tube 26 of the needle shield 24 relaxes passively from its collapsed state and again shields the needle tip 22 from inadvertent needle stick contact with the patient's eye 40 or any other object.

When injection devices of the present disclosure are packaged for distribution to healthcare providers, the passive needle shield prevents needle sticks during the entire syringe use cycle, e.g., no piercing of the sterilized packaging during shipment and storage, no inadvertent needle contact during subsequent clinician handling of the device during a patient treatment procedure, or thereafter during used syringe disposal. This eliminates the need to package the syringe with a separate, external needle shield for shipment that otherwise must be removed by a clinician at the treatment site. Elimination of the need to remove a separate, external needle shield saves clinician time and avoids potential loss of needle sterility by decreasing needle exposure to ambient air or contact with non-sterile objects. Another potential needle stick is avoided during transport of the used syringe directly to a disposal receptacle and/or by the need to replace the separate, external needle shield prior to syringe disposal.

In clinical use of the syringes of the present disclosure, potentially provide one or more of the following advantages, jointly or severally. There is no needle tip exposure except during the actual patient injection. The abutment of the needle shield's contact surface against the patient's tissue at the injection site assures better stability during the injection procedure because the syringe moves with the patient tissue as the clinician initiates needle piercing of the tissue at the site. This is a great benefit in ocular injection procedures to avoid inadvertent needle contact with the patient's cornea. Coordination of tissue and needle movement during injection initiation prevents patient injury, such as the aforementioned potential cornea injury during ocular injection procedures. The clinician also benefits by reducing the risk of accidental needle stick injury during the injection process Similar advantages are provided when syringe injection device of the present disclosure is used for dermatological injection administrations, neonatal injections, biologics injections, spinal injections, and others general forms of medical injection.

Reference throughout this specification to "one embodiment," "certain embodiments," "various embodiments," "one or more embodiments" or "an embodiment" means that a particular feature, structure, material, or characteristic described in connection with the embodiment is included in at least one embodiment of the disclosure. Thus, the appearances of the phrases such as "in one or more embodiments," "in certain embodiments," "in various embodiments," "in one embodiment" or "in an embodiment" in various places throughout this specification are not necessarily referring to the same embodiment of the disclosure. Furthermore, the particular features, structures, materials, or characteristics may be combined in any suitable manner in one or more embodiments.

Although the disclosure herein provided a description with reference to particular embodiments, it is to be understood that these embodiments are merely illustrative of the principles and applications of the disclosure. It will be apparent to those skilled in the art that various modifications and variations can be made to the present disclosure without departing from the spirit and scope thereof. Thus, it is intended that the present disclosure include modifications and variations that are within the scope of the appended claims and their equivalents.

Further aspects of the invention are:
1. A medical injection device, comprising:
   a syringe including a barrel that defines a barrel cavity with a proximal open end, a barrel tip on a distal end of the barrel, a plunger inserted in a proximal open end of the barrel that is translatable in the barrel cavity, the plunger defining a plunger stopper on a distal end thereof;
   a needle hub coupled to the barrel tip, the needle hub coupled to a needle that defines a needle tip, oriented distal the needle hub, the needle extending a first axial length relative to the needle hub;
   the barrel cavity, barrel tip, needle hub and needle in fluid communication with each other; and
   a collapsible needle shield coupled to the distal end of the syringe barrel, the needle shield extending distally from the needle hub and circumscribing the needle, the needle shield having a contact surface on a distal axial end thereof and an aperture formed therein that is coaxial with the needle;

   wherein, when the needle shield is in a relaxed, non-collapsed state, its contact surface extends axially from the needle hub a second axial length that is longer than the first axial length of the needle, so that the needle tip is oriented proximal to and shielded by the contact surface; and
   wherein, when the needle shield is in a collapsed state, its contact surface extends axially from the needle hub a third axial length that is shorter than the first axial length of the needle, so that the needle tip is exposed and extends distally through the aperture of the contact surface.
2. The injection device of aspect 1, the collapsible needle shield comprising a flexible bellows tube having a proximal axial end coupled to the needle hub, a distal axial end defining the contact surface and its aperture, and an internal cannula circumscribing the needle.
3. The injection device of aspect 2, further comprising the flexible bellows tube and a cup-shaped, flexible contact surface formed as a polymer unistructure.
4. The injection device of aspect 1, the needle shield further comprising a cup-shaped, flexible contact surface.
5. The injection device of aspect 1, the collapsible needle shield comprising:
   a needle shield hub coupled to the distal end of the syringe barrel;
   first and second hinge assemblies, each respectively having a proximal link coupled to the needle shield hub, a link coupled to a distal end of the proximal link, and a distal link coupled to the link hinge distal the proximal link; and
   the contact surface coupled to the respective distal ends of the distal links of the first and second hinge assemblies, with the aperture of the contact surface oriented coaxially with the needle.
6. The injection device of aspect 5, further comprising a tubular cover coupled to a proximal side of the contact surface and oriented coaxial with the needle, a cannular passage of the tube in communication with the aperture for passage of the needle therethrough when the collapsible needle shield is in a collapsed state.
7. The injection device of aspect 6, the collapsible needle shield comprising a polymer unistructure.
8. The injection device of aspect 1, further comprising the collapsible needle shield coupled to the needle hub.
9. A method for injecting a patient with the injection device of aspect 1, comprising:
   contacting a desired site on a patient's body with the contact surface of the injection device;
   advancing the syringe barrel to depress the body site with the contact surface, thereby stabilizing the aperture of the contact surface relative to the body site;
   pivoting the syringe barrel after stabilizing the contact surface and its aperture, thereby orienting a central axis of the needle in a desired angular position relative to the body site;
   further advancing the syringe barrel, thereby collapsing the needle shield and piercing the body site with the needle tip to a desired depth within the body;
   injecting the patient with fluid contained in the syringe barrel's cavity by advancing the plunger; and
   withdrawing the syringe from the patient's body thereby causing the needle shield to relax from its collapsed state and again shield the needle tip.
10. The injection device of aspect 1, further comprising the contact surface is an ocular contact surface having a surface profile for abutment against a sclera of a patient's eye.
11. The injection device of aspect 10, further comprising a flexible ocular contact surface that conforms to surface profile of a sclera of a patient's eye when abutted against the sclera.
12. The injection device of aspect 11, the flexible ocular contact surface comprising a cup-shaped skirt.
13. A method for injecting a patient's eye with the injection device of aspect 10, comprising:
   contacting a desired site on a sclera of a patient's eye with the ocular contact surface of the injection device;
   advancing the syringe barrel to depress the sclera with the ocular contact surface, thereby stabilizing the ocular contact surface and its aperture relative to the sclera;
   pivoting the syringe barrel after stabilizing the aperture, thereby orienting a central axis of the needle in a desired angular position relative to the sclera;
   further advancing the syringe barrel, thereby collapsing the needle shield and piercing the sclera with the needle tip to a desired depth within the eye;
   injecting the intra ocular region of the patient's eye with fluid contained in the syringe barrel's cavity by advancing the plunger; and
   withdrawing the syringe from the patient's eye, thereby causing the needle shield to relax from its collapsed state and again shield the needle tip.
14. A medical injection device, comprising:
   a syringe including a barrel that defines a barrel cavity with a proximal open end, a barrel tip on a distal end of the barrel, a plunger inserted in a proximal open end of the barrel that is translatable in the barrel cavity, the plunger defining a plunger stopper on a distal end thereof;
   a needle hub coupled to the barrel tip, the needle hub coupled to a needle that defines a needle tip, oriented distal the needle hub, the needle extending a first axial length relative to the needle hub;
   the barrel cavity, barrel tip, needle hub and needle in fluid communication with each other; and
   a flexible bellows tube forming a collapsible needle shield, the bellows tube defining an internal cannula, a proximal axial end of the bellows tube coupled directly to the needle hub, the bellows tube extending distally from the needle hub and circumscribing the needle within its internal cannula, a distal axial end of the bellows tube coupled to a cup-shaped skirt, whose distal axial end defines a contact surface, and an aperture formed in the cup-shaped skirt that is in communication with the internal cannula and that is coaxial with the needle;
      wherein, when the flexible bellows is in a relaxed, non-collapsed state, the contact surface of the skirt extends axially from the needle hub a second axial length that is longer than the first axial length of the needle, so that the needle tip is oriented proximal to and shielded by the ocular contact surface; and
      wherein, the flexible bellows is in a collapsed state, the contact surface of the skirt extends axially from the needle hub a third axial length that is shorter than the first axial length of the needle, so that the needle tip is exposed and extends distally through the aperture of the ocular contact surface.
15. The ocular injection device of aspect 14, the distal face of the cup-shaped skirt capable of adhering and/or suction anchoring to a patient's eye sclera and stabilizing the syringe.
16. A method for injecting a patient with the injection device of aspect 14, comprising:
   contacting a desired site on a patient's body with the contact surface of the injection device;
   advancing the syringe barrel to depress the body site with the contact surface, thereby stabilizing the aperture of the contact surface relative to the body site;
   pivoting the syringe barrel after stabilizing the contact surface and its aperture, thereby orienting a central axis of the needle in a desired angular position relative to the body site;
   further advancing the syringe barrel, thereby collapsing the needle shield and piercing the body site with the needle tip to a desired depth within the body;
   injecting the patient with fluid contained in the syringe barrel's cavity by advancing the plunger; and
   withdrawing the syringe from the patient's body thereby causing the needle shield to relax from its collapsed state and again shield the needle tip.
17. A method for injecting a patient's eye with the medical injection device of aspect 14, comprising:
   contacting a desired site on a sclera of a patient's eye with the distal face of cup-shaped skirt, contact surface of the injection device;
   advancing the syringe barrel to depress the sclera with the contact surface, thereby stabilizing the aperture of the contact surface relative to the sclera;
   pivoting the syringe barrel after stabilizing the aperture, thereby orienting a central axis of the needle in a desired angular position relative to the sclera;
   further advancing the syringe barrel, thereby collapsing the needle shield and piercing the sclera with the needle tip to a desired depth within the eye;
   injecting the intra ocular region of the patient's eye with fluid contained in the syringe barrel's cavity by advancing the plunger; and
   withdrawing the syringe from the patient's eye, thereby causing the needle shield to relax from its collapsed state and again shield the needle tip.
18. A medical injection device, comprising:
   a syringe including a barrel that defines a barrel cavity with a proximal open end, a barrel tip on a distal end of the barrel, a plunger inserted in a proximal open end of the barrel that is translatable in the barrel cavity, the plunger defining a plunger stopper on a distal end thereof;
   a needle hub coupled to the barrel tip, the needle hub coupled to a needle that defines a needle tip, oriented distal the needle hub, the needle extending a first axial length relative to the needle hub;
   the barrel cavity, barrel tip, needle hub and needle in fluid communication with each other;
   a collapsible needle shield, including:
      a needle shield hub directly coupled to the needle hub;
      first and second hinge assemblies, each respectively having a proximal link coupled to the needle shield hub, a link coupled to a distal end of the proximal link, and a distal link coupled to the link hinge distal the proximal link;
      a contact surface coupled to the respective distal ends of the distal links of the first and second hinge assemblies, the contact surface defining a through aperture that is oriented coaxially with the needle; and
      a tubular cover coupled to a proximal side of the contact surface and oriented coaxial with the needle, a cannular passage of the tube in communication with the aperture for passage of the needle therethrough;

      wherein, when the collapsible needle shield is in a relaxed, non-collapsed state, the contact surface extends axially from the needle hub a second axial length that is longer than the first axial length of the needle, so that the needle tip is oriented proximal to and shielded by the contact surface and the tubular cover; and
      wherein, when the collapsible needle shield is in a collapsed state, the contact surface extends axially from the needle hub a third axial length that is shorter than the first axial length of the needle, so that the needle tip is exposed and extends distally through the aperture of the contact surface.
19. The medical injection device of aspect 18, the collapsible needle shield comprising a polymer unistructure.
20. A method for injecting a patient with the injection device of aspect 18, comprising:
   contacting a desired site on a patient's body with the contact surface of the injection device;
   advancing the syringe barrel to depress the body site with the contact surface, thereby stabilizing the aperture of the contact surface relative to the body site;
   pivoting the syringe barrel after stabilizing the contact surface and its aperture, thereby orienting a central axis of the needle in a desired angular position relative to the body site;
   further advancing the syringe barrel, thereby collapsing the needle shield and piercing the body site with the needle tip to a desired depth within the body;
   injecting the patient with fluid contained in the syringe barrel's cavity by advancing the plunger; and
   withdrawing the syringe from the patient's body thereby causing the needle shield to relax from its collapsed state and again shield the needle tip.
21. A method for injecting a patient's eye with the medical injection device of aspect 18, comprising:
   contacting a desired site on a sclera of a patient's eye with the contact surface of the injection device;
   advancing the syringe barrel to depress the sclera with the contact surface, thereby stabilizing the aperture of the ocular contact surface relative to the sclera;
   pivoting the syringe barrel after stabilizing the aperture, thereby orienting a central axis of the needle in a desired angular position relative to the sclera;
   further advancing the syringe barrel, thereby collapsing the needle shield and piercing the sclera with the needle tip to a desired depth within the eye;
   injecting the intra ocular region of the patient's eye with fluid contained in the syringe barrel's cavity by advancing the plunger; and
   withdrawing the syringe from the patient's eye, thereby causing the needle shield to relax from its collapsed state and again shield the needle tip.

## Claims

1. A medical injection device, comprising:
a syringe including a barrel that defines a barrel cavity with a proximal open end, a barrel tip on a distal end of the barrel, a plunger inserted in a proximal open end of the barrel that is translatable in the barrel cavity, the plunger defining a plunger stopper on a distal end thereof;
a needle hub coupled to the barrel tip, the needle hub coupled to a needle that defines a needle tip, oriented distal the needle hub, the needle extending a first axial length relative to the needle hub;
the barrel cavity, barrel tip, needle hub and needle in fluid communication with each other; and
a collapsible needle shield coupled to the distal end of the syringe barrel, the needle shield extending distally from the needle hub and circumscribing the needle, the needle shield having a contact surface on a distal axial end thereof and an aperture formed therein that is coaxial with the needle;
wherein, when the needle shield is in a relaxed, non-collapsed state, its contact surface extends axially from the needle hub a second axial length that is longer than the first axial length of the needle, so that the needle tip is oriented proximal to and shielded by the contact surface; and
wherein, when the needle shield is in a collapsed state, its contact surface extends axially from the needle hub a third axial length that is shorter than the first axial length of the needle, so that the needle tip is exposed and extends distally through the aperture of the contact surface.

2. The injection device of claim 1, the collapsible needle shield comprising a flexible bellows tube having a proximal axial end coupled to the needle hub, a distal axial end defining the contact surface and its aperture, and an internal cannula circumscribing the needle.

3. The injection device of claim 2, further comprising the flexible bellows tube and a cup-shaped, flexible contact surface formed as a polymer unistructure.

4. The injection device of claim 1, the needle shield further comprising a cup-shaped, flexible contact surface.

5. The injection device of claim 1, the collapsible needle shield comprising:
a needle shield hub coupled to the distal end of the syringe barrel;
first and second hinge assemblies, each respectively having a proximal link coupled to the needle shield hub, a link coupled to a distal end of the proximal link, and a distal link coupled to the link hinge distal the proximal link; and
the contact surface coupled to the respective distal ends of the distal links of the first and second hinge assemblies, with the aperture of the contact surface oriented coaxially with the needle.

6. The injection device of claim 5, further comprising a tubular cover coupled to a proximal side of the contact surface and oriented coaxial with the needle, a cannular passage of the tube in communication with the aperture for passage of the needle therethrough when the collapsible needle shield is in a collapsed state.

7. The injection device of claim 6, the collapsible needle shield comprising a polymer unistructure.

8. The injection device of claim 1, further comprising the collapsible needle shield coupled to the needle hub.

9. The injection device of claim 1, further comprising the contact surface is an ocular contact surface having a surface profile for abutment against a sclera of a patient's eye.

10. The injection device of claim 9, further comprising a flexible ocular contact surface that conforms to surface profile of a sclera of a patient's eye when abutted against the sclera.

11. The injection device of claim 10, the flexible ocular contact surface comprising a cup-shaped skirt.

12. A medical injection device, comprising:
a syringe including a barrel that defines a barrel cavity with a proximal open end, a barrel tip on a distal end of the barrel, a plunger inserted in a proximal open end of the barrel that is translatable in the barrel cavity, the plunger defining a plunger stopper on a distal end thereof;
a needle hub coupled to the barrel tip, the needle hub coupled to a needle that defines a needle tip, oriented distal the needle hub, the needle extending a first axial length relative to the needle hub;
the barrel cavity, barrel tip, needle hub and needle in fluid communication with each other; and
a flexible bellows tube forming a collapsible needle shield, the bellows tube defining an internal cannula, a proximal axial end of the bellows tube coupled directly to the needle hub, the bellows tube extending distally from the needle hub and circumscribing the needle within its internal cannula, a distal axial end of the bellows tube coupled to a cup-shaped skirt, whose distal axial end defines a contact surface, and an aperture formed in the cup-shaped skirt that is in communication with the internal cannula and that is coaxial with the needle;
wherein, when the flexible bellows is in a relaxed, non-collapsed state, the contact surface of the skirt extends axially from the needle hub a second axial length that is longer than the first axial length of the needle, so that the needle tip is oriented proximal to and shielded by the ocular contact surface; and
wherein, the flexible bellows is in a collapsed state, the contact surface of the skirt extends axially from the needle hub a third axial length that is shorter than the first axial length of the needle, so that the needle tip is exposed and extends distally through the aperture of the ocular contact surface.

13. The ocular injection device of claim 12, the distal face of the cup-shaped skirt capable of adhering and/or suction anchoring to a patient's eye sclera and stabilizing the syringe.

14. A medical injection device, comprising:
a syringe including a barrel that defines a barrel cavity with a proximal open end, a barrel tip on a distal end of the barrel, a plunger inserted in a proximal open end of the barrel that is translatable in the barrel cavity, the plunger defining a plunger stopper on a distal end thereof;
a needle hub coupled to the barrel tip, the needle hub coupled to a needle that defines a needle tip, oriented distal the needle hub, the needle extending a first axial length relative to the needle hub;
the barrel cavity, barrel tip, needle hub and needle in fluid communication with each other;
a collapsible needle shield, including:
a needle shield hub directly coupled to the needle hub;
first and second hinge assemblies, each respectively having a proximal link coupled to the needle shield hub, a link coupled to a distal end of the proximal link, and a distal link coupled to the link hinge distal the proximal link;
a contact surface coupled to the respective distal ends of the distal links of the first and second hinge assemblies, the contact surface defining a through aperture that is oriented coaxially with the needle; and
a tubular cover coupled to a proximal side of the contact surface and oriented coaxial with the needle, a cannular passage of the tube in communication with the aperture
for passage of the needle therethrough;
wherein, when the collapsible needle shield is in a relaxed, non-collapsed state, the contact surface extends axially from the needle hub a second axial length that is longer than the first axial length of the needle, so that the needle tip is oriented proximal to and shielded by the contact surface and the tubular cover; and
wherein, when the collapsible needle shield is in a collapsed state, the contact surface extends axially from the needle hub a third axial length that is shorter than the first axial length of the needle, so that the needle tip is exposed and extends distally through the aperture of the contact surface, preferably wherein:
the collapsible needle shield comprising a polymer unistructure.

15. A method for injecting:
- a patient with the injection device of claim 1, comprising:
contacting a desired site on a patient's body with the contact surface of the injection device;
advancing the syringe barrel to depress the body site with the contact surface, thereby stabilizing the aperture of the contact surface relative to the body site;
pivoting the syringe barrel after stabilizing the contact surface and its aperture, thereby orienting a central axis of the needle in a desired angular position relative to the body site;
further advancing the syringe barrel, thereby collapsing the needle shield and piercing the body site with the needle tip to a desired depth within the body;
injecting the patient with fluid contained in the syringe barrel's cavity by advancing the plunger; and
withdrawing the syringe from the patient's body thereby causing the needle shield to relax from its collapsed state and again shield the needle tip; or
- a patient's eye with the injection device of claim 9, comprising:
contacting a desired site on a sclera of a patient's eye with the ocular contact surface of the injection device;
advancing the syringe barrel to depress the sclera with the ocular contact surface, thereby stabilizing the ocular contact surface and its aperture relative to the sclera;
pivoting the syringe barrel after stabilizing the aperture, thereby orienting a central axis of the needle in a desired angular position relative to the sclera;
further advancing the syringe barrel, thereby collapsing the needle shield and piercing the sclera with the needle tip to a desired depth within the eye;
injecting the intra ocular region of the patient's eye with fluid contained in the syringe barrel's cavity by advancing the plunger; and
withdrawing the syringe from the patient's eye, thereby causing the needle shield to relax from its collapsed state and again shield the needle tip; or
- a patient with the injection device of claim 12, comprising:
contacting a desired site on a patient's body with the contact surface of the injection device;
advancing the syringe barrel to depress the body site with the contact surface, thereby stabilizing the aperture of the contact surface relative to the body site;
pivoting the syringe barrel after stabilizing the contact surface and its aperture, thereby orienting a central axis of the needle in a desired angular position relative to the body site;
further advancing the syringe barrel, thereby collapsing the needle shield and piercing the body site with the needle tip to a desired depth within the body;
injecting the patient with fluid contained in the syringe barrel's cavity by advancing the plunger; and
withdrawing the syringe from the patient's body thereby causing the needle shield to relax from its collapsed state and again shield the needle tip; or
- a patient's eye with the medical injection device of claim 12, comprising:
contacting a desired site on a sclera of a patient's eye with the distal face of cup-shaped skirt, contact surface of the injection device;
advancing the syringe barrel to depress the sclera with the contact surface, thereby stabilizing the aperture of the contact surface relative to the sclera;
pivoting the syringe barrel after stabilizing the aperture, thereby orienting a central axis of the needle in a desired angular position relative to the sclera;
further advancing the syringe barrel, thereby collapsing the needle shield and piercing the sclera with the needle tip to a desired depth within the eye;
injecting the intra ocular region of the patient's eye with fluid contained in the syringe barrel's cavity by advancing the plunger; and
withdrawing the syringe from the patient's eye, thereby causing the needle shield to relax from its collapsed state and again shield the needle tip; or
- a patient with the injection device of claim 14, comprising:
contacting a desired site on a patient's body with the contact surface of the injection device;
advancing the syringe barrel to depress the body site with the contact surface, thereby stabilizing the aperture of the contact surface relative to the body site;
pivoting the syringe barrel after stabilizing the contact surface and its aperture, thereby orienting a central axis of the needle in a desired angular position relative to the body site;
further advancing the syringe barrel, thereby collapsing the needle shield and piercing the body site with the needle tip to a desired depth within the body;
injecting the patient with fluid contained in the syringe barrel's cavity by advancing the plunger; and
withdrawing the syringe from the patient's body thereby causing the needle shield to relax from its collapsed state and again shield the needle tip; or
- a patient's eye with the medical injection device of claim 14, comprising:
contacting a desired site on a sclera of a patient's eye with the contact surface of the injection device;
advancing the syringe barrel to depress the sclera with the contact surface, thereby stabilizing the aperture of the ocular contact surface relative to the sclera;
pivoting the syringe barrel after stabilizing the aperture, thereby orienting a central axis of the needle in a desired angular position relative to the sclera;
further advancing the syringe barrel, thereby collapsing the needle shield and piercing the sclera with the needle tip to a desired depth within the eye;
injecting the intra ocular region of the patient's eye with fluid contained in the syringe barrel's cavity by advancing the plunger; and
withdrawing the syringe from the patient's eye, thereby causing the needle shield to relax from its collapsed state and again shield the needle tip.
